# EUROPÄISCHE PATENTANMELDUNG

(11) **EP 3 093 042 A2**
(43) Veröffentlichungstag der Anmeldung: **16.11.2016**
(21) Anmeldenummer: 16161306.2
(22) Anmeldetag: 21.03.2016
(51) Int. Cl.: A61N 1/05, A61M 25/04

(54) **IMPLANTIERBARES GEBOGENES FORMGEBUNGSTEIL ZUR ÄUSSEREN FORMUNG EINER IMPLANTIERBAREN ELEKTRODENLEITUNG ODER EINES KATHETERS**

(30) Priorität: 20.04.2015 US 201562149672 P
(71) Anmelder: BIOTRONIK SE & Co. KG, 12359 Berlin (DE)
(72) Erfinder: Jadwizak, Detmar, 15537 Erkner (DE); Fründt, Carsten, 13057 Berlin (DE); Kaiser, Dajana, 12247 Berlin (DE); Hillebrand, Gordon, 12157 Berlin (DE)
(74) Vertreter: Galander, Marcus

(57) **Zusammenfassung**

Implantierbares gebogenes Formgebungsteil zur äußeren Formung einer implantierbaren Elektrodenleitung oder eines Katheters, wobei das Formgebungsteil ein durchgehendes Lumen zum Durchgang eines Abschnitts der Elektrodenleitung oder des Katheters hat, wobei das Formgebungsteil einen extrudierten Schlauch aus zwei jeweils über ein vorbestimmtes Wandungs-Segment koextrudierten Materialien mit unterschiedlichem Schrumpfungsverhalten oder einen Abschnitt eines extrudierten Spiralschlauches aufweist, wobei eines der koextrudierten Materialien oder das Material des Spiralschlauches eine hohe Shore-Härte, insbesondere von 60 Shore oder mehr, aufweist.

## Beschreibung

Die Erfindung betrifft ein implantierbares gebogenes Formgebungsteil zur äußeren Formung einer implantierbaren Elektrodenleitung oder eines Katheters, wobei das Formgebungsteil ein durchgehendes erstes Lumen zum Durchgang eines Abschnitts der Elektrodenleitung oder des Katheters hat. Sie betrifft des Weiteren eine Elektrodenleitungs- oder Katheteranordnung, die mit mindestens einem solchen Formgebungsteil gebildet ist.

Implantierte medizinische Elektrodenleitungen (kurz auch als "Elektroden" bezeichnet) müssen im Körper des Patienten an einer geeigneten Stelle positioniert und in dieser Position auch hinreichend dauerhaft fixiert werden, um die gewünschten therapeutischen Wirkungen zuverlässig und dauerhaft zu erzielen. Es hat daher seit der Einführung der ersten implantierbaren Herzschrittmacher eine Vielzahl unterschiedlicher Entwicklungen zur Lösung dieser Aufgabe gegeben.

Zur positionsgetreuen Fixierung von Elektrodenleitungen in Gefäßabschnitten bzw. relativ engen Hohlorganen wurden bereits vor langem Elektrodenleitungen mit eingeprägten Krümmungen im distalen Endbereich vorgeschlagen, die sich nach der Implantation und dem Entfernen des Führungsdrahtes (Guide Wire) oder des Mandrins (Stylet) aufgrund Ihres gekrümmten Verlaufes gewissermaßen zwischen gegenüberliegenden Wandungen des Gefäßes oder Hohlorgans verspannen. Hierdurch kann eine recht verlässliche und auch langzeitstabile Positionsfixierung erreicht werden. Ein Beispiel einer solchen Konstruktion ist beschrieben in US 5,925,073.

In diesem Zusammenhang sind auch Elektrodenleitungen mit speziellen inneren Strukturen entwickelt worden, mit denen eine über den Längsverlauf variable Steifigkeit bzw. Biegsamkeit realisiert werden soll; vgl. hierzu etwa US 6,556,873 B1 oder EP 2 024 014 B1.

Speziell sogenannte CRT-Elektroden werden in Koronarsinusgefäßen im Bereich des linken Ventrikels implantiert. Dazu besitzen diese Elektroden eine "passive" Fixierung der oben erwähnten Art, indem der distale Bereich der Elektrode mit einer oder mehreren Krümmungen versehen ist. Bei der Implantation wird diese Krümmung durch einen innen liegenden Mandrin gerade gestellt. Wird dieser Mandrin zurückgezogen, übt die sich wieder krümmende Elektrode Kraft auf die Gefäßinnenwand aus und verankert somit die Elektrode an ihrem gewünschten Ort.

Aus technologischer Sicht sind im Wesentlichen folgende Realisierungsmöglichkeiten einer solchen Lösung bekannt und in Gebrauch:
1. Erzeugen einer Elektrodenkrümmung durch Glühen einer im Leitungskörper liegenden MP35N Wendel.
2. Erzeugen einer Elektrodenkrümmung durch mechanische Verformung (Kaltverformung) einer im Leitungskörper liegenden Wendel. Das wird vorzugsweise bei Coradialwendeln angewandt, da hier jeder Einzeldraht eine Isolationsschicht besitzt, deren Schmelzpunkt unter der Glühtemperatur der Wendel liegt, sodass ein Glühprozess nicht angewendet werden kann.
3. Erzeugen einer Elektrodenkrümmung durch thermisches Verformen von Kunststof fisolationsschläuchen.
4. Erzeugen einer Elektrodenkrümmung durch ein in gekrümmter Form spritzgegossenes Silikonteil.
5. Erzeugen einer Elektrodenkrümmung durch ein Silikonspritzgussteil mit Spannband.

Alle diese Lösungen haben gewisse Nachteile, von denen hier die folgenden genannt werden sollen:
Zu 1.: Bei Verwendung einer ETFE beschichteten Coradialwendel kann kein Glühverfahren angewandt werden. Um mehrere Pole in einem kleinen Durchmesser zu realisieren, ist die Verwendung einer solchen Coradialwendel aber erforderlich.
Zu 2.: Allein durch eine mechanische Verformung der Wendel werden nicht der gewünschte Krümmungswinkel und die gewünschte Krümmungskraft erzeugt.
Zu 3.: Bei Verwendung von Silikon kann keine nachträgliche, thermische Verformung realisiert werden. Silikon ist ein endvernetztes Material, welches sich nach dem Vulkanisierungsprozess nicht mehr umformen lässt. Im distalen Bereich einer CRT-Elektrode ist die Verwendung von Silikon von großem Vorteil, da Flexibilität und Dauerfestigkeit einem Kunststoffmaterial für diese Anwendung überlegen sind.
Zu 4.: Ein Silikonspritzgussteil, welches krumm gespritzt ist, erzeugt nicht den gewünschten Krümmungswinkel und die gewünschte Krümmungskraft (Rückstellkraft).
Zu 5.: Aufgrund des kleinen Durchmessers einer Elektrodenleitung steht für ein Spannband eine nur sehr kleine Wandstärke zur Verfügung. Des Weiteren ist ein Silikonspritzgussteil mit Spannband nur in einem komplizierten und aufwendigen Herstellungsverfahren mit verhältnismäßig hohem Fehlerpotential herstellbar.

Es ist daher Aufgabe der Erfindung, eine verbesserte Lösung zur Realisierung einer in einem Gefäß oder relativ engen Hohlorgan passiv fixierbaren Elektrodenleitung (oder auch eines Katheters) bereitzustellen, die insbesondere zuverlässig und dauerhaft funktioniert, bei der Implantation leicht zu handhaben ist und auch unter Kostenaspekten vertretbar ist.

Es ist, gemäß grundlegenden Überlegungen der Erfinder, ein Silikonteil zu entwickeln, welches bei einem kleinen Durchmesser, geringer Wandstärke, geringer Länge und einfachem Herstellungsverfahren die Funktion der Krümmung einer Coradialwendel und damit die Fixierung der Elektrode in einem Gefäß oder Hohlorgan (speziell einem im Koronargefäß) übernimmt. Dieses Silikonteil muss flexibel, geradestellbar und dauerfest sein sowie eine möglichst hohe Rückstellkraft aufweisen.

Diese Aufgabe wird gelöst durch ein implantierbares gebogenes Formgebungsteil mit den Merkmalen des Anspruchs 1. Zweckmäßige Fortbildungen des Erfindungsgedankens sind Gegenstand der abhängigen Ansprüche. Es werden des Weiteren eine Elektrodenleitungsanordnung mit den Merkmalen des Anspruchs 6 und eine Katheteranordnung mit den Merkmalen des Anspruchs 9 bereitgestellt.

Die Erfindung schließt die Überlegung ein, zur Aufprägung einer gewünschten Krümmung auf eine Elektrodenleitung oder einen Katheterschlauch ein separates Formgebungsteil bereitzustellen, welches in technologisch einfacher und somit kostengünstiger Weise im Wesentlichen einen extrudierten Silikonschlauch oder Abschnitt eines solchen darstellt. Damit ein solcher Silikonschlauch oder -schlauchabschnitt seine Formgebungsfunktion erfüllen kann, muss er selbst im Gebrauchszustand gebogen oder gekrümmt sein und bei einer Deformation (in Richtung auf einen gestreckten Zustand) ein hinreichend hohes Rückstellmoment erzeugen können, dass die zu formende, im Lumen des Formgebungsteils aufgenommene Elektrodenleitung (bzw. ein Katheterschlauch) das Rückstellen in die gebogene Gestalt nicht verhindert, sondern vielmehr beim Rückstellen in die gekrümmte Form "mitgenommen" wird.

Gemäß einem ersten Aspekt der Erfindung ist dazu vorgesehen, dass das Formgebungsteil einen extrudierten Schlauch aus zwei jeweils über ein vorbestimmtes Wandungs-Segment koextrudierten Materialien mit unterschiedlichem Schrumpfungsverhalten aufweist, wobei eines der koextrudierten Materialien eine hohe Shore-Härte, insbesondere von 60 Shore oder mehr, aufweist. Die Materialien mit unterschiedlichem Schrumpfungsverhalten bewirken bei dem der Koextrusion nachgelagerten Temperschritt, dass der Schlauch im fertigen Zustand gekrümmt ist, und die Einbindung mindestens eines Materials mit relativ hoher Shore-Härte sorgt für ein hinreichend hohes Rückstellmoment, im oben erwähnten Sinne.

Gemäß einem relativ unabhängigen Aspekt der Erfindung stellt das Formgebungsteil einen Abschnitt eines extrudierten Spiralschlauches aus einem Material mit hoher Shore-Härte, wiederum insbesondere von 60 Shore oder mehr dar. Die Herstellung von spiraligen (helikalen) Schläuchen durch Extrusionsverfahren ist eine etablierte Technik und auch bei Silikonschläuchen anwendbar. Im Kontext der vorliegenden Erfindung ist zur Erzielung einer hinreichend großen "kraftverstärkenden" Wirkung bzw. Rückstellkraft auf eine hinreichend hohe Steifigkeit bzw. Shore-Härte des Schlauchmaterials zu achten. Es versteht sich, dass diese für die konkrete Realisierung auf die Eigenschaften der zu fixierenden Elektrodenleitung bzw. eines Katheterschlauches abzustimmen ist. Dies gilt selbstverständlich auch für die geometrischen Dimensionen (Durchmesser und Länge) des Schlauchabschnittes sowie sinnvollerweise auch für den Helix-Durchmesser des als Basis dienenden Spiralschlauches.

In einer Ausführung der Erfindung ist das Formgebungsteil ausgebildet als im Gebrauchszustand U-förmiges oder V-förmiges Teil mit verrundeter Spitze. Als Gebrauchszustand ist der Zustand mit im Formgebungsteil aufgenommener Elektroden- oder Katheterleitung, oder versteifenden Führungsdraht, zu verstehen. Je nach Länge des Formgebungsteils kann dessen eigene Grundform (ohne Elektroden- oder Katheterleitung) durchaus ein Kreissegment von mehr als 180° umfassen, ja sogar ansatzweise eine Wendel- bzw. Helixform haben.

In einer aus derzeitiger Sicht bevorzugten Ausführung weist das Formgebungsteil einen Silikonschlauch oder Silikonschlauchabschnitt auf. Grundsätzlich ist die Erfindung allerdings auch mit anderen geeigneten Materialien, insbesondere mit für den medizinischen Einsatz zugelassenen und bewährten Polymeren, möglich.

In einer weiteren Ausführung hat das Formgebungsteil Enden, die mit einem Material beliebiger Shore-Härte, z.B. zwischen 30 und 80 Shore umspritzt sind. Hierdurch sind Lumen-Endabschnitte mit derart aufgeweitetem Durchmesser gebildet, dass Platz für eine innenliegende Klebstoffschicht geschaffen ist. Bevorzugt sind diese Enden des Formgebungsteils im Wesentlichen gestreckt ausgebildet. Für den praktischen Einsatz der in Rede stehenden Elektrodenleitungs- oder Katheteranordnungen ist, schon unter Zulassungs-Aspekten, eine feste, stoffschlüssige Verbindung des Formgebungsteils mit einem vorbestimmten Abschnitt der Elektroden- oder Katheterleitung (insbesondere angrenzend an einen Elektrodenpol) zu schaffen. Um dies ohne äußerliche (und somit im Einsatz hinderliche) Durchmesservergrößerung zu erreichen, ist an der Innenwandung des Formgebungsteils Platz für eine (insbesondere ringförmige) Klebstoffschicht zu schaffen.

In einer Ausgestaltung des o.a. ersten Aspekts der Erfindung nehmen die koextrudierten Materialien des extrudierten Schlauches jeweils ein halbzylindrisches Wandungs-Segment ein. Grundsätzlich können die beiden Koextrudierten Materialien aber auch unterschiedliche Anteile der Wandung des Formgebungsteils bilden, und die konkrete Wahl der jeweiligen Anteile kann von der Härte des jeweiligen Materials, den Unterschieden im Schrumpfungsverhalten und natürlich auch den Parametern der zu formenden Elektrodenleitung oder des Katheterschlauches abhängen.

In einer Ausführung der vorgeschlagenen Elektrodenleitungsanordnung ist vorgesehen, dass das Formgebungsteil einen Abschnitt zwischen zwei Elektrodenpolen vollständig umgibt, derart, dass ein erstes Ende des Formgebungsteils direkt an einen ersten Elektrodenpol anstößt und ein zweites Ende des Formgebungsteils direkt an einen zweiten Elektrodenpol anstößt. Hiermit wird den weiter oben angesprochenen Anforderungen an die dauerhafte Zuverlässigkeit, z.B. hinsichtlich Isolation, Zugkraft, Dauerfestigkeit die sich auch in Zulassungserfordernissen ausdrücken, Rechnung getragen.

Um eine sichere Verbindung zwischen dem Formgebungsteil und Elektrodenpolen zu gewährleisten, ist es notwendig gestufte innere Absätze an den Enden des Formgebungsteiles für die erforderliche Klebeschicht zu haben. Die Elektrodenpole besitzen ebenfalls einen Absatz, sodass ausreichend Klebefläche für eine sichere Zugkraftübertragung, Isolation und Dauerfestigkeit vorhanden ist. Allein eine stirnseitige Anbindung ohne stufige Ansätze könnte diese Anforderungen nicht erfüllen.

In weiteren Ausführungen der Elektrodenleitungsanordnung sind zwei oder mehr U-förmige oder V-förmige Formgebungsteile in Längsrichtung hintereinander auf der Elektrodenleitung mit entgegengerichteter Krümmung derart angeordnet, dass ein distaler Endabschnitt der Elektrodenleitung insgesamt ein im Wesentlichen S-förmiger, Z-förmiger, J-förmiger, wellenförmiger oder zickzackförmiger Verlauf jeweils auch 3-dimensional aufgeprägt hat.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A bis 1F: Darstellungen zu einem ersten Ausführungsbeispiel der Erfindung,
- Fig. 2A bis 2G: Darstellungen zu einem zweiten Ausführungsbeispiel der Erfindung,
- Fig. 3A und 3B: perspektivische Darstellungen zweier Ausführungen einer erfindungsgemäßen Elektrodenleitungsanordnung.

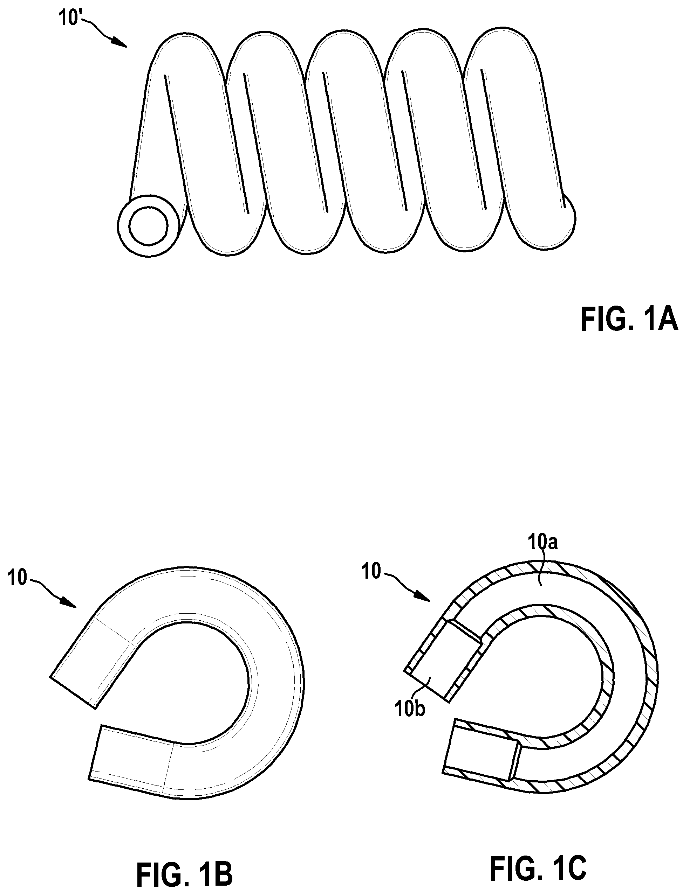

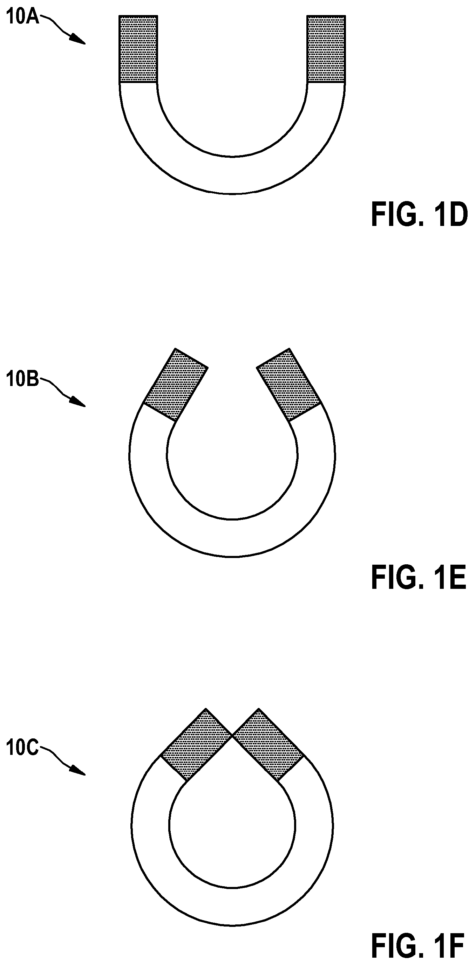

Fig. 1A zeigt in einer perspektivischen Darstellung einen Abschnitt eines extrudierten Silikon-Spiralschlauches 10' als Vorprodukt der Herstellung eines erfindungsgemäßen Formgebungsteiles; Fig. 1B und 1C zeigen ein Formgebungsteil 10 in einer Seitenansicht bzw. Längsschnittdarstellung, und die Figuren 1D bis 1F zeigen verschieden geformte Formgebungsteile 10A, 10B, 10C.

Der in Fig. 1A gezeigte Spiralschlauch wird durch Extrusion eines geradlinig gestreckten Silikonschlauches mit nachgelagerter Spiralformgebung oder durch Extrusion eines Spiralschlauches mittels einer freidrehend gelagerten Düse gemäß einem an sich bekannten Verfahren hergestellt. Es wird hierbei ein Silikonmaterial mit hoher Shore-Härte, z.B. 70 oder 80 Shore genutzt, um die mit einer solch hohen Steifigkeit bzw. Shore-Härte verbundene hohe Krümmungskraft für ein aus dem Spiralschlauch hergestelltes Formgebungsteil zu nutzen.

Das in Fig. 1B und 1C dargestellte Formgebungsteil 10 wird durch Ablängen (Ausschneiden) eines Spiralschlauch-Abschnittes vorbestimmter Länge aus dem Spiralschlauch 10' nach Fig. 1A und anschließendes Anspritzen von geradlinigen Endabschnitten in einem Spritzgießwerkzeug hergestellt. Wie in Fig. 1C zu erkennen, werden die Endabschnitte des Formgebungsteils 10 hierbei mit einem Durchmesser ihres Lumens 10b angespritzt, der gegenüber dem Innendurchmesser des Silikonschlauchabschnittes (Durchmesser des Lumens 10a) etwas vergrößert ist. Diese Endabschnitte vergrößerten Durchmessers schaffen Platz für eine bei der späteren Montage des Formgebungsteils über einer Elektrodenleitung oder einem Katheterschlauch erforderliche Klebstoffschicht. Zur Erzeugung der Endabschnitte wird eine Spritzgussmasse mit nach dem konkreten Anwendungsfall einzustellender Shore-Härte genutzt, beispielsweise ein Material mit einer Shore-Härte zwischen 30 und 80 Shore. Alternativ kann ein Endabschnitt mit vergrößertem Durchmesser auch ohne Spritzgussprozess hergestellt werden, und zwar durch Fräsen der Endabschnittkontur des zugeschnittenen Spiralschlauchabschnitts.

Wie in Fig. 1C bis 1F skizzenartig dargestellt, können durch Einsatz eines geeigneten Vorprodukts (Spiralschlauches) mit speziell gewähltem Durchmesser und durch Wahl der Länge des hieraus ausgeschnittenen Schlauchabschnittes Formgebungsteile mit weitgehend wählbarer Form dargestellt werden. In Fig. 1D bis 1F sind die Endabschnitte mit erweitertem Innendurchmesser jeweils etwas dunkler dargestellt, aber nicht gesondert bezeichnet. Es versteht sich, dass neben den hier skizzierten Varianten, bei denen sich der Kreissegment-Abschnitt des Formgebungsteils über einen Winkelbereich zwischen ca. 180° und ca. 270° erstreckt, auch Varianten mit noch geringerer, aber auch mit noch größerer WinkelErstreckung des Kreissegment-Abschnitts realisieren lassen. Im Übrigen ist zu erwähnen, dass bei Nutzung von Silikon mit hoher Shore-Härte der finale Krümmungswinkel bzw. Kreissegment-Winkel umso größer wird, je kleiner der Durchmesser und die Steigung des als Vorprodukt dienenden Spiralschlauches gewählt werden.

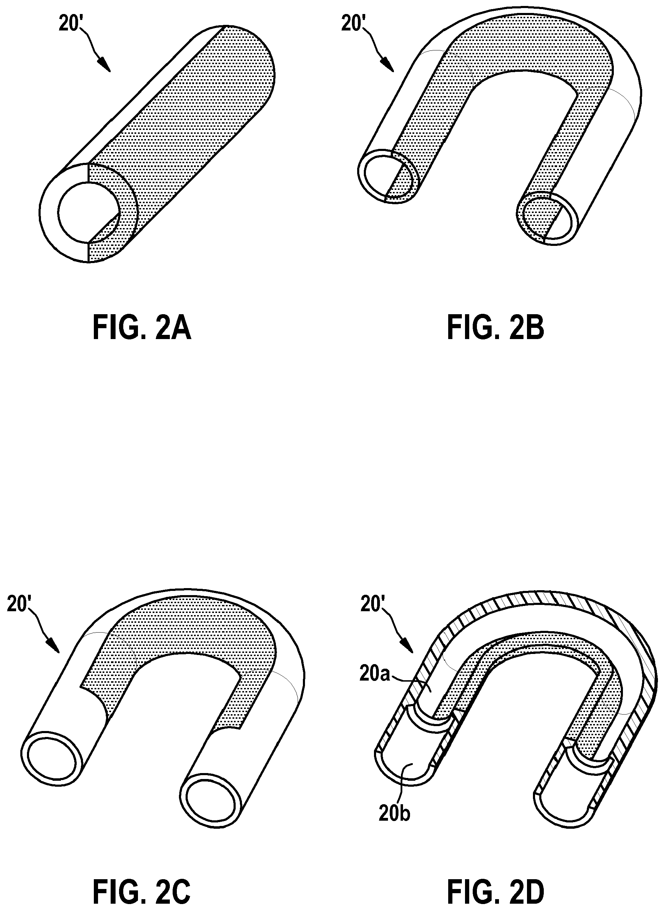

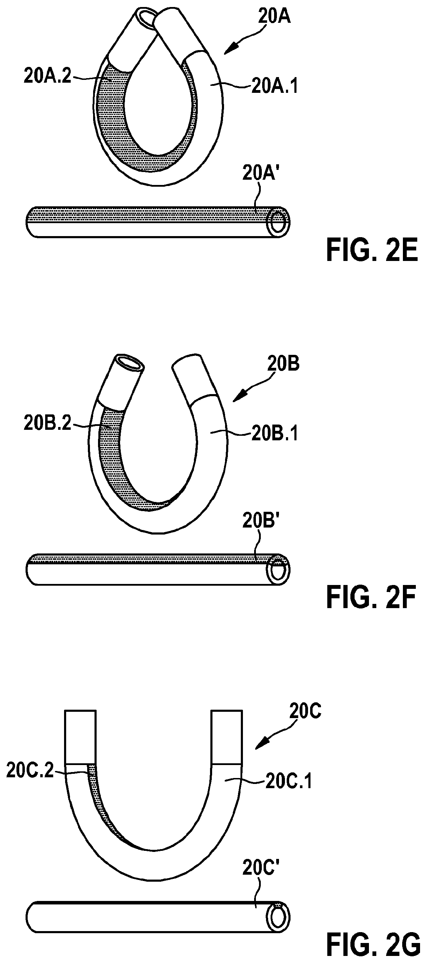

Fig. 2A zeigt schematisch einen langgestreckten Silikonschlauchabschnitt und Fig. 2B den Silikonschlauchabschnitt in nach einem Temperschritt U-förmig gebogenen Zustand; Fig. 2C und 2D zeigen ein auf dieser Basis hergestelltes Formgebungsteil in einer perspektivischen Darstellung bzw. perspektivischen Längsschnittdarstellung; und die Figuren 2E bis 2G zeigen schematisch Form-Varianten eines solchen Formgebungsteiles, jeweils zusammen mit dem als Vorprodukt dienenden geradlinig gestreckten Schlauchabschnitt. Es ist darauf hinzuweisen, dass das Vorprodukt wie auch das resultierende Formgebungsteil aus zwei verschiedenen Silikonmaterialien mit unterschiedlicher Shore-Härte und hieraus resultierendem unterschiedlichem Schrumpfungsverhalten gebildet sind, welches für die Ausbildung der Krümmung des Vorprodukts und des Formgebungsteils ursächlich ist.

Die in Fig. 2B gezeigte U-förmige Gestalt des Silikonschlauch-Vorproduktes 20' ist also durch Tempern und hierbei gegebenes unterschiedliches Schrumpfungsverhalten des aus zwei Silikonmaterialien gemäß einer Konfiguration wie in Fig. 2E und 2G geradlinig koextrudierten primären Silikonschlauches entstanden. An beide Enden des U-förmig gebogenen Vorprodukts wird ein Endabschnitt mit einem gegenüber dem Lumen 20a des primären Schlauches erweitertem Lumen 20b angespritzt. Hierdurch wird, wie bei der vorstehend beschriebenen ersten Ausführung, ein ringförmiger Spalt für ein Klebstoffreservoir geschaffen.

In Fig. 2E bis 2G sind verschiedene Varianten der Zusammensetzung des koextrudierten primären Schlauches und verschiedene sich daraus ergebende Formen des auf dieser Basis hergestellten Formgebungsteiles gezeigt. Fig. 2E zeigt eine Variante, bei der die beiden Silikonmaterialien 20.A1, 20.A2 im Vorprodukt 20A' jeweils einen Winkelbereich von 180° der Schlauchwandung einnehmen, woraus sich ein Formgebungsteil 20A ergibt, dessen kreissegmentförmiger Mittenabschnitt einen Kreissegment-Winkelbereich von nahezu 270° abdeckt. Gemäß Fig. 2F hat ein Silikonmaterial 20.B2 einen Anteil von deutlich weniger als 180° an der Wandung des Vorprodukts 20b', und nach dem Tempern und dem Anspritzen der Endabschnitte ergibt sich ein Formgebungsteil 20B, dessen kreissegmentförmiger zentraler Abschnitt sich über einen Winkelbereich von ca. 220° erstreckt. Fig. 2G schließlich zeigt einen gestreckten Schlauchabschnitt 20C', bei dem die beiden Silikonmaterialien 20.C1, 20C2 einen sehr stark unterschiedlichen Anteil in der Schlauchwandung haben, woraus letztlich (nach Tempern und Anspritzen von Endabschnitten) ein Formgebungsteil mit annähernder U-Form, also einer Winkelerstreckung des kreissegmentförmigen Abschnitts von ca. 180°, resultiert.

Es versteht sich, dass neben der geeigneten Wahl des Anteils der beiden Materialkomponenten beim Koextrudieren des als Vorprodukt dienenden Schlauchabschnitts auch der Unterschied in der Shore-Härte und im Schrumpfungsverhalten wichtig für die Ausbildung einer gewünschten Endform des Formgebungsteils ist. Dem Fachmann sind die entsprechenden Materialgrößen und sich nach dem Tempern ergebenden Formen gebräuchlicher medizinischer Silikone bekannt, so dass von genaueren Angaben hierzu an dieser Stelle abgesehen werden kann.

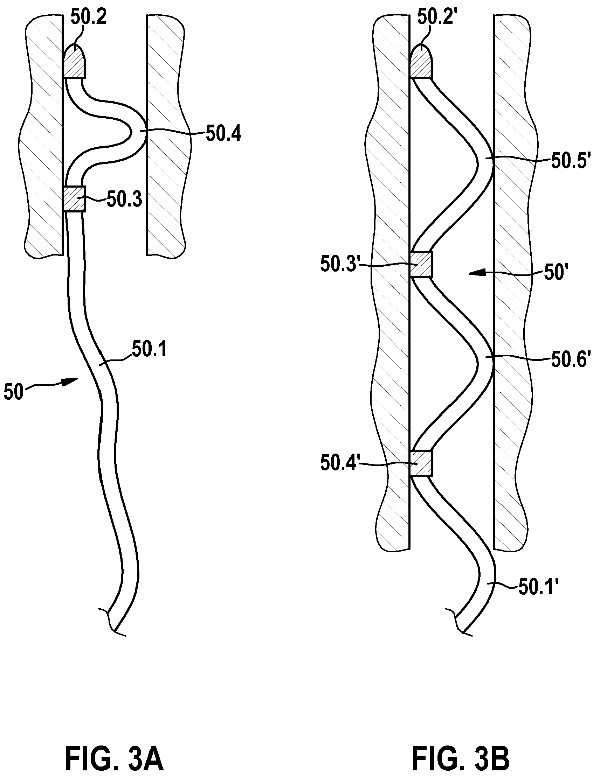

Fig. 3A und 3B zeigen zwei Konfigurationen von Elektrodenleitungsanordnungen, die mit Formgebungsteilen der vorstehend beschriebenen Art realisiert werden können. Fig. 3A zeigt eine Elektrodenleitungsanordnung 50 mit einer Elektrodenleitung (Elektrode) 50.1, die eine Spitzenelektrode 50.2 und eine Ringelektrode 50.3 als Elektrodenpole hat und die durch eine zwischen den beiden Elektrodenpolen 50.2, 50.3 auf die Leitung aufgeschrumpftes Formgebungsteil 50.4 im Endabschnitt V-förmig verformt ist. Hierdurch verspannt sich die Elektrodenleitungsanordnung 50 zwischen den gegenüberliegenden Wandungen eines Gefäßes, was wiederum in erwünschter Weise dazu führt, dass die Elektrodenpole 50.2 und 50.3 zuverlässigen Wandkontakt haben und somit ihre Stimulations- und/oder Sensing-Aufgabe dauerhaft zuverlässig erfüllen können.

Fig. 3B zeigt als Abwandlung dieser Konfiguration eine weitere Elektrodenleitungsanordnung 50', die eine dreipolige Elektrodenleitung 50.1' mit den Elektrodenpolen 50,2', 50.3' und 50.4' und zwei Formgebungsteile 50.5', 50.6' umfasst. Beide Formgebungsteile sind jeweils zwischen zweien der drei Elektrodenpole um die entsprechenden Abschnitte der Elektrode 50.1' gelegt und dort mit der Elektrode dicht verklebt. Es ist zu erkennen, dass die Elektrodenanordnung bei dieser Ausführungsform eine im Endabschnitt insgesamt wellenförmige Gestalt angenommen hat, wobei diese Gestalt wiederum das erwünschte Ergebnis hat, dass alle Elektrodenpole 50.2', 50.3' und 50.4' zuverlässigen Wandkontakt haben.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Implantierbares gebogenes Formgebungsteil (10; 10A; 10B; 10C; 20; 20A; 20B; 20C) zur äußeren Formung einer implantierbaren Elektrodenleitung (50) oder eines Katheters, wobei das Formgebungsteil ein durchgehendes Lumen (10a; 20a) zum Durchgang eines Abschnitts der Elektrodenleitung oder des Katheters hat,
wobei das Formgebungsteil einen extrudierten Schlauch (20') aus zwei jeweils über ein vorbestimmtes Wandungs-Segment koextrudierten Materialien mit unterschiedlichem Schrumpfungsverhalten oder einen Abschnitt eines extrudierten Spiralschlauches (10') aufweist, wobei eines der koextrudierten Materialien oder das Material des Spiralschlauches eine hohe Shore-Härte, insbesondere von 60 Shore oder mehr, aufweist.

2. Formgebungsteil nach Anspruch 1, ausgebildet als im Gebrauchszustand U-förmiges oder V-förmiges Teil mit verrundeter Spitze.

3. Formgebungsteil nach Anspruch 1 oder 2, welches einen Silikonschlauch (20') oder Silikonschlauchabschnitt (10') aufweist.

4. Formgebungsteil nach einem der vorangehenden Ansprüche, mit Enden, die mit einem Material geringerer Shore-Härte, insbesondere von 40 Shore oder weniger, umspritzt sind, wodurch Lumen-Endabschnitte (20b) mit derart aufgeweitetem Durchmesser gebildet sind, dass Platz für eine innenliegende Klebstoffschicht geschaffen ist.

5. Formgebungsteil nach einem der vorangehenden Ansprüche, wobei die koextrudierten Materialien des extrudierten Schlauches (20') jeweils ein halbzylindrisches Wandungs-Segment einnehmen.

6. Elektrodenleitungsanordnung (50; 50') mit einer implantierbaren Elektrodenleitung und mindestens einem Formgebungsteil (50.4; 50.5'; 50.6') nach einem der vorangehenden Ansprüche, welches einen distalen Abschnitt der Elektrodenleitung (50.1; 50.1') umgibt.

7. Elektrodenleitungsanordnung nach Anspruch 6, wobei das Formgebungsteil (50.4; 50.5', 50.6') einen Abschnitt zwischen zwei Elektrodenpolen (50.2, 50.3; 50.2', 50.3', 50.4') vollständig umgibt, derart, dass ein erstes Ende des Formgebungsteils direkt an einen ersten Elektrodenpol anstößt und ein zweites Ende des Formgebungsteils direkt an einen zweiten Elektrodenpol anstößt.

8. Elektrodenleitungsanordnung nach Anspruch 6 oder 7, wobei zwei oder mehr U-förmige oder V-förmige Formgebungsteile (50.5', 50.6') in Längsrichtung hintereinander auf der Elektrodenleitung (50.1'), optional mit entgegengerichteter Krümmung derart angeordnet sind, dass ein distaler Endabschnitt der Elektrodenleitung insgesamt einen im Wesentlichen S-förmigen, Z-förmigen, J-förmigen, wellenförmigen oder zickzackförmigen Verlauf aufgeprägt hat.

9. Katheteranordnung mit einem Katheter und mindestens einem Formgebungsteil (10) nach einem der Ansprüche 1 bis 5.
